# EUROPEAN PATENT APPLICATION

(11) **EP 4 119 575 A1**
(43) Date of publication of application: **18.01.2023**
(21) Application number: 21768221.0
(22) Date of filing: 10.02.2021
(51) Int. Cl.: C07K 16/10, A61K 39/215, A61K 39/395, A61P 31/14, C07K 7/08, C07K 14/165, G01N 33/53, G01N 33/569, G01N 33/577, C12N 15/50

(54) **EPITOPE OF ANTIBODY AGAINST STRUCTURAL PROTEIN OF SARS-COV-2, ANTIBODY REACTING WITH EPITOPE, METHOD FOR DETECTING SARS-COV-2 USING ANTIBODY, DETECTION KIT FOR SARS-COV-2 CONTAINING ANTIBODY, METHOD FOR DETECTING ANTI-SARS-COV-2 ANTIBODY CONTAINING POLYPEPTIDE OF EPITOPE, DETECTION KIT FOR ANTI-SARS-COV-2 ANTIBODY CONTAINING POLYPEPTIDE OF EPITOPE, VACCINE FOR SARS-COV-2 CONTAINING POLYPEPTIDE OF EPITOPE, AND THERAPEUTIC AGENT FOR SARS-COV-2 INFECTION CONTAINING ANTIBODY**

(30) Priority: 10.03.2020 JP 2020040960
(71) Applicant: Denka Company Limited, Tokyo 103-8338 (JP)
(72) Inventor: OGASAWARA Shinya, Tokyo 103-8338 (JP)
(74) Representative: Hoffmann Eitle
(86) International application number: PCT/JP2021/005011
(87) International publication number: WO 2021/181994

(57) **Abstract**

The present invention relates to a monoclonal antibody or an antigen-binding fragment thereof, wherein the monoclonal antibody or the antigen-binding fragment thereof reacts with a structural protein of SARS-CoV-2 specifically, and the structural protein of SARS-CoV-2 is at least one selected from the group consisting of S-protein, N-protein, M-protein, and E-protein and a hapten, wherein the hapten that reacts with an antibody that reacts with a protein of SARS-CoV-2 specifically, and the protein of SARS-CoV-2 is at least one selected from the group consisting of S-protein, N-protein, M-protein, and E-protein.

## Description

### Technical Field

The present invention relates to an epitope of an antibody against a structural protein of SARS-CoV-2, an antibody reacting with the epitope, a method for detecting SARS-CoV-2 using the antibody, a detection kit for SARS-CoV-2 comprising the antibody, a method for detecting an anti-SARS-CoV-2 antibody comprising the polypeptide of the epitope, a detection kit for the anti-SARS-CoV-2 antibody comprising the polypeptide of the epitope, a vaccine for SARS-CoV-2 comprising the polypeptide of the epitope, and a therapeutic agent for a SARS-CoV-2 infection comprising the antibody.

### Background Art

Coronaviruses are enveloped viruses having a positive-sense single-stranded RNA genome and a helically symmetric nucleocapsid. Coronaviruses that infect humans include betacoronavirus, and it is known that especially MERS-coronavirus (MERS-CoV) and SARS-coronavirus (SARS-CoV) cause severe respiratory symptoms.

A novel coronavirus (SARS-CoV-2) broke out in China in 2019, and the infection has continuously spread to Southeast Asia, the Middle East, Europe, and the like around East Asia as of February, 2020. The development of a rapid simple detection method in addition to a therapy and a vaccine against SARS-CoV-2 is therefore urgently necessary to detect SARS-CoV-2 at an early stage and to suppress infection spread.

Although the antigenicity and candidate epitopes of SARS-CoV-2 have been reported after the outbreak of SARS-CoV-2 (for example, Non Patent Literature 1), the candidate epitopes are for the development of a vaccine against SARS-CoV-2, and an epitope suitable for detecting SARS-CoV-2 and an antibody that recognizes the epitope have not been reported yet.

### Citation List

### Non Patent Literature

Non Patent Literature 1: Suresh Kumar, Preprints, doi: https://www.preprints.org/manuscript/202002.0071/v1

### Summary of Invention

### Technical Problem

An object of the present invention is to provide an antibody for detecting SARS-CoV-2, a method for detecting SARS-CoV-2 using the antibody, and a detection kit comprising the antibody. An object of the present invention is to provide an antibody for simultaneously detecting a human infectious coronavirus such as MERS-CoV or SARS-CoV in addition to SARS-CoV-2 that cause severe respiratory symptoms, a method for detecting SARS-CoV-2 and a human infectious coronavirus simultaneously using the antibody, and a detection kit comprising the antibody.

### Solution to Problem

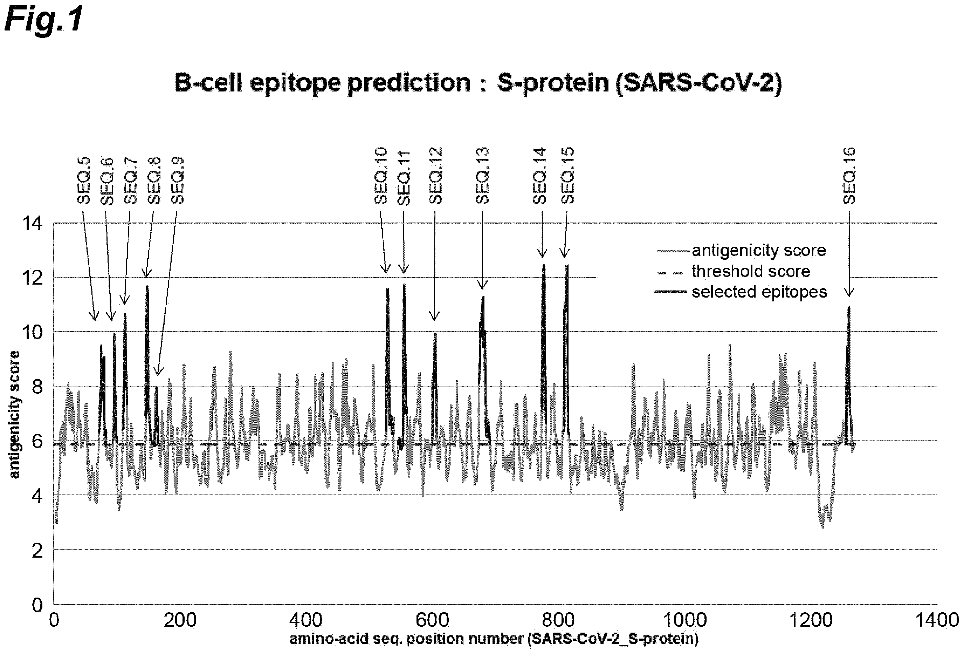

Although a plurality of algorithms for predicting antigenicities have been reported until now (for example, Chou PY, Fasman GD. 1978. Prediction of the secondary structure of proteins from their amino acid sequence. Adv Enzymol Relat Areas Mol Biol 47:45-148), these algorithms had a problem that these algorithms included contradictory indices, and these algorithms were therefore lacking in accuracy in antigenicity prediction by individual algorithms. The present inventors have made improvements to enable the combination of these algorithms for enhancing antigenic accuracy, found that the viral antigenicity is strong in regions having specific amino acid sequences in structural proteins constituting SARS-CoV-2, and the specific amino acid sequences are useful as an epitope (polypeptide) that an antibody for detecting SARS-CoV-2 recognizes from the antigen intensity scores of all the structural protein molecules constituting SARS-CoV-2 obtained using the improved algorithm (Figures 1 to 4), and completed the present invention.

More specifically, the present invention provides a monoclonal antibody or an antigen-binding fragment thereof that specifically reacts with the structural protein of SARS-CoV-2 and a detection kit for SARS-CoV-2 comprising the antibody. Here, the structural protein of SARS-CoV-2 is at least one protein selected from the group consisting of S-protein, N-protein, M-protein, and E-protein.

The present invention provides a method for detecting SARS-CoV-2 in a specimen including contacting a monoclonal antibody or an antigen-binding fragment thereof that specifically reacts with a structural protein of SARS-CoV-2 with a specimen and detecting SARS-CoV-2 by an immunological measuring method. In the method for detecting SARS-CoV-2 in a specimen, a sandwich method using at least two monoclonal antibodies or antigen-binding fragments thereof for each structural protein is preferable.

The present invention provides a detection kit for SARS-CoV-2 and a human infectious coronavirus other than SARS-CoV-2 (preferably MERS-CoV and/or SARS-CoV) comprising a monoclonal antibody or an antigen-binding fragment thereof that specifically reacts with a structural protein of SARS-CoV-2 and a structural protein of a human infectious coronavirus other than SARS-CoV-2 (preferably MERS-CoV and/or SARS-CoV) and the antibody. Here, the structural protein of SARS-CoV-2 is at least one protein selected from the group consisting of S-protein, N-protein, M-protein, and E-protein, and the structural protein of the human infectious coronavirus other than SARS-CoV-2 is at least one protein selected from the group consisting of S-protein, N-protein, M-protein, and E-protein.

The present invention provides an epitope (polypeptide), wherein an antibody for detecting SARS-CoV-2 recognizes the epitope (polypeptide). The present invention furthermore provides a method for detecting an anti-SARS-CoV-2 antibody comprising the polypeptide of the epitope, a detection kit for the anti-SARS-CoV-2 antibody comprising the polypeptide of the epitope, and a vaccine for SARS-CoV-2 comprising the polypeptide of the epitope, and a therapeutic agent for a SARS-CoV-2 infection comprising the antibody.

### Advantageous Effects of Invention

According to the present invention, an antibody for detecting SARS-CoV-2, a method for detecting SARS-CoV-2 using the antibody, and a detection kit comprising the antibody can be provided. According to the present invention, an antibody for simultaneously detecting a coronavirus such as MERS-CoV or SARS-CoV in addition to SARS-CoV-2 that cause severe respiratory symptoms, and a detection kit comprising the antibody can be provided. According to the present invention, an epitope (polypeptide), wherein the antibody for detecting SARS-CoV-2 recognizes the epitope (polypeptide) can furthermore be provided.

### Brief Description of Drawings

[Figure 1] Figure 1 is a graph obtained by scoring the antigen intensity of the whole S-protein molecule of SARS-CoV-2.
[Figure 2] Figure 2 is a graph obtained by scoring the antigen intensity of the whole N-protein molecule of SARS-CoV-2.
[Figure 3] Figure 3 is a graph obtained by scoring the antigen intensity of the whole M-protein molecule of SARS-CoV-2.
[Figure 4] Figure 4 is a graph obtained by scoring the antigen intensity of the whole E-protein molecule of SARS-CoV-2.
[Figure 5a] Figure 5a is a graph showing the specific reactivity with a polypeptide having the sequence of SEQ ID NO: 5 at the time of immunization against the polypeptide.
[Figure 5b] Figure 5b is a graph showing the specific reactivity with a polypeptide having the sequence of SEQ ID NO: 6 at the time of immunization against the polypeptide.
[Figure 5c] Figure 5c is a graph showing the specific reactivity with a polypeptide having the sequence of SEQ ID NO: 7 at the time of immunization against the polypeptide.
[Figure 5d] Figure 5d is a graph showing the specific reactivity with a polypeptide having the sequence of SEQ ID NO: 8 at the time of immunization against the polypeptide.
[Figure 5e] Figure 5e is a graph showing the specific reactivity with a polypeptide having the sequence of SEQ ID NO: 9 at the time of immunization against the polypeptide.
[Figure 5f] Figure 5f is a graph showing the specific reactivity with a polypeptide having the sequence of SEQ ID NO: 10 at the time of immunization against the polypeptide.
[Figure 5g] Figure 5g is a graph showing the specific reactivity with a polypeptide having the sequence of SEQ ID NO: 11 at the time of immunization against the polypeptide.
[Figure 5h] Figure 5h is a graph showing the specific reactivity with a polypeptide having the sequence of SEQ ID NO: 13 at the time of immunization against the polypeptide.
[Figure 5i] Figure 5i is a graph showing the specific reactivity with a polypeptide having the sequence of SEQ ID NO: 15 at the time of immunization against the polypeptide.
[Figure 5j] Figure 5j is a graph showing the specific reactivity with a polypeptide having the sequence of SEQ ID NO: 16 at the time of immunization against the polypeptide.
[Figure 5k] Figure 5k is a graph showing the specific reactivity with a polypeptide having the sequence of SEQ ID NO: 18 at the time of immunization against the polypeptide.
[Figure 5l] Figure 5Iis a graph showing the specific reactivity with a polypeptide having the sequence of SEQ ID NO: 19 at the time of immunization against the polypeptide.
[Figure 5m] Figure 5m is a graph showing the specific reactivity with a polypeptide having the sequence of SEQ ID NO: 20 at the time of immunization against the polypeptide.
[Figure 5n] Figure 5n is a graph showing the specific reactivity with a polypeptide having the sequence of SEQ ID NO: 21 at the time of immunization against the polypeptide.
[Figure 5o] Figure 5o is a graph showing the specific reactivity with a polypeptide having the sequence of SEQ ID NO: 23 at the time of immunization against the polypeptide.
[Figure 5p] Figure 5p is a graph showing the specific reactivity with a polypeptide having the sequence of SEQ ID NO: 24 at the time of immunization against the polypeptide.
[Figure 5q] Figure 5q is a graph showing the specific reactivity with a polypeptide having the sequence of SEQ ID NO: 25 at the time of immunization against the polypeptide.
[Figure 5r] Figure 5r is a graph showing the specific reactivity with a polypeptide having the sequence of SEQ ID NO: 26 at the time of immunization against the polypeptide.
[Figure 5s] Figure 5s is a graph showing the specific reactivity with a polypeptide having the sequence of SEQ ID NO: 27 at the time of immunization against the polypeptide.
[Figure 5t] Figure 5t is a graph showing the specific reactivity with a polypeptide having the sequence of SEQ ID NO: 30 at the time of immunization against the polypeptide.
[Figure 5u] Figure 5u is a graph showing the specific reactivity with a polypeptide having the sequence of SEQ ID NO: 31 at the time of immunization against the polypeptide.
[Figure 5v] Figure 5v is a graph showing the specific reactivity with a polypeptide having the sequence of SEQ ID NO: 32 at the time of immunization against the polypeptide.
[Figure 5w] Figure 5w is a graph showing the specific reactivity with a polypeptide having the sequence of SEQ ID NO: 33 at the time of immunization against the polypeptide.
[Figure 5x] Figure 5x is a graph showing the specific reactivity with a polypeptide having the sequence of SEQ ID NO: 34 at the time of immunization against the polypeptide.
[Figure 6] Figure 6 is a graph showing the reactivity of a total of 24 antibodies obtained in Example 1 with a SARS-CoV-2 inactivated antigen.
[Figure 7] Figure 7 is a graph showing the results obtained by analyzing epitopes of an antibody present in antiserum obtained by immunization against a SARS-CoV-2 inactivated antigen.
[Figure 8] Figure 8 is a graph showing the results obtained by analyzing epitopes of an antibody present in antiserum obtained by immunization against a recombinant N-protein (full-length) described in SEQ ID NO: 2.
[Figure 9] Figure 9 is the graph showing the reactivities of antibodies in antiserums obtained in Examples 3 and 4 with N-protein epitope sequences having SEQ ID NOS: 17 to 25.

### Description of Embodiments

[Antibody against SARS-CoV-2, method for detecting SARS-CoV-2 using the antibody, and detection kit comprising the antibody]

SARS-CoV-2 is a virus of betacoronavirus mainly comprising S-protein (surface protein), N-protein (nucleocapsid protein), M-protein (membrane protein), and E-protein (envelope protein). A monoclonal antibody according to the present embodiment specifically reacts with at least one protein selected from the group consisting of the main structural proteins constituting SARS-CoV-2, namely S-protein, N-protein, M-protein, and E-protein.

"Specifically" in the present embodiment means that, in a liquid system in which protein and a monoclonal antibody according to the present embodiment are mixed, the antibody does not cause antigen-antibody reaction with protein components other than the proteins of SARS-CoV-2 at a detectable level, or even though the antibody causes some binding or association reaction, the antibody causes only reaction clearly weaker than an antigen-antibody reaction of the antibody with a protein of SARS-CoV-2.

The S-protein (surface protein; hereinafter also called merely "S-protein") of SARS-CoV-2 consists of the sequence of 1273 amino acids set forth by SEQ ID NO: 1 representatively. The S-protein may be a protein consisting of the amino acid sequence of SEQ ID NO: 1, or may be a protein having 90% or more (preferably 95% or more) sequence identity with SEQ ID NO: 1.

A monoclonal antibody according to the present embodiment that specifically reacts with the S-protein of SARS-CoV-2 binds to an amino acid region of the S-protein corresponding to amino acid positions from 66 to 85 (SEQ ID NO: 5), from 87 to 107 (SEQ ID NO: 6), from 105 to 131 (SEQ ID NO: 7), from 140 to 157 (SEQ ID NO: 8), from 155 to 170 (SEQ ID NO: 9), from 522 to 541 (SEQ ID NO: 10), from 538 to 562 (SEQ ID NO: 11), from 595 to 611 (SEQ ID NO: 12), from 670 to 693 (SEQ ID NO: 13), from 761 to 797 (SEQ ID NO: 14), from 802 to 818 (SEQ ID NO: 15), or from 1235 to 1273 (SEQ ID NO: 16). In Figure 1, obtained by scoring the antigen intensity of the whole S-protein molecule, these amino acid regions have high antigenicity scores as compared with a threshold score, and are polypeptides having a sequence consisting of at least 16 amino acids.

The monoclonal antibody according to the present embodiment that specifically reacts with the S-protein of SARS-CoV-2 preferably binds to an amino acid region of the S-protein corresponding to amino acid positions from 71 to 83, from 89 to 103, from 107 to 127, from 143 to 154, from 158 to 167, from 524 to 538, from 546 to 560, from 598 to 608, from 672 to 691, from 772 to 796, from 806 to 816, or from 1238 to 1269. In Figure 1, obtained by scoring the antigen intensity of the whole S-protein molecule, these amino acid regions have higher antigenicity scores as compared with a threshold score, and are polypeptides having a sequence consisting of at least 10 amino acids.

The monoclonal antibody according to the present embodiment that specifically reacts with the S-protein more preferably binds to an amino acid region of the S-protein corresponding to amino acid positions from 73 to 79, from 93 to 99, from 109 to 115, from 145 to 152, from 160 to 165, from 526 to 531, from 553 to 558, from 599 to 607, from 674 to 684, from 773 to 795, from 808 to 814, or from 1257 to 1261. In Figure 1, obtained by scoring the antigen intensity of the whole S-protein molecule, these amino acid regions have the highest antigenicity scores as compared with a threshold score, and are polypeptides having a sequence consisting of at least 5 amino acids.

The N-protein (nucleocapsid protein; hereinafter also called merely "N-protein") of SARS-CoV-2 consists of the sequence of 419 amino acids set forth by SEQ ID NO: 2.

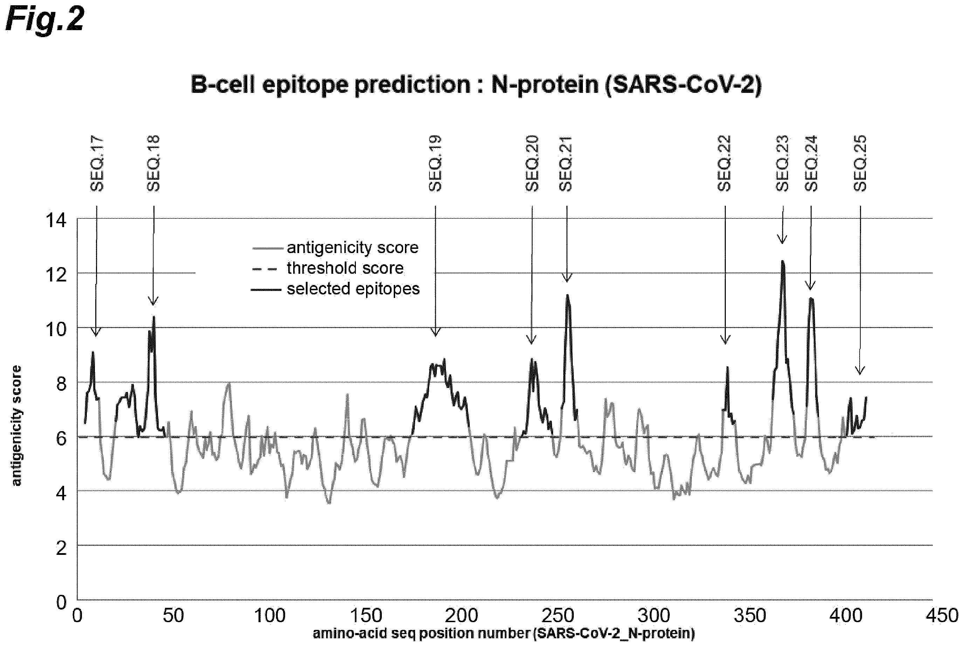

A monoclonal antibody according to the present embodiment that specifically reacts with the N-protein binds to an amino acid region of the N-protein corresponding to amino acid positions from 1 to 18 (SEQ ID NO: 17), from 19 to 49 (SEQ ID NO: 18), from 174 to 207 (SEQ ID NO: 19), from 230 to 252 (SEQ ID NO: 20), from 247 to 267 (SEQ ID NO: 21), from 336 to 350 (SEQ ID NO: 22), from 362 to 379 (SEQ ID NO: 23), from 377 to 395 (SEQ ID NO: 24), or from 401 to 419 (SEQ ID NO: 25). In Figure 2, obtained by scoring the antigen intensity of the whole N-protein molecule, these amino acid regions have high antigenicity scores as compared with a threshold score, and are polypeptides having a sequence consisting of at least 15 amino acids.

The monoclonal antibody according to the present embodiment that specifically reacts with the N-protein preferably binds to an amino acid region of the N-protein corresponding to amino acid positions from 1 to 12, from 20 to 48, from 175 to 206, from 234 to 250, from 254 to 263, from 338 to 348, from 366 to 377, from 381 to 391, or from 403 to 417. In Figure 2, obtained by scoring the antigen intensity of the whole N-protein molecule, these amino acid regions have higher antigenicity scores as compared with a threshold score, and are polypeptides having a sequence consisting of at least 10 amino acids.

The monoclonal antibody according to the present embodiment that specifically reacts with the N-protein more preferably binds to an amino acid region of the N-protein corresponding to amino acid positions from 4 to 11, from 27 to 41, from 185 to 196, from 237 to 247, from 256 to 261, from 340 to 347, from 367 to 375, from 384 to 390, or from 406 to 415. In Figure 2, obtained by scoring the antigen intensity of the whole N-protein molecule, these amino acid regions have the highest antigenicity scores as compared with a threshold score, and are polypeptides having a sequence consisting of at least 6 amino acids.

The M-protein (membrane protein; hereinafter also called merely "M-protein") of SARS-CoV-2 consists of the sequence of 222 amino acids set forth by SEQ ID NO: 3.

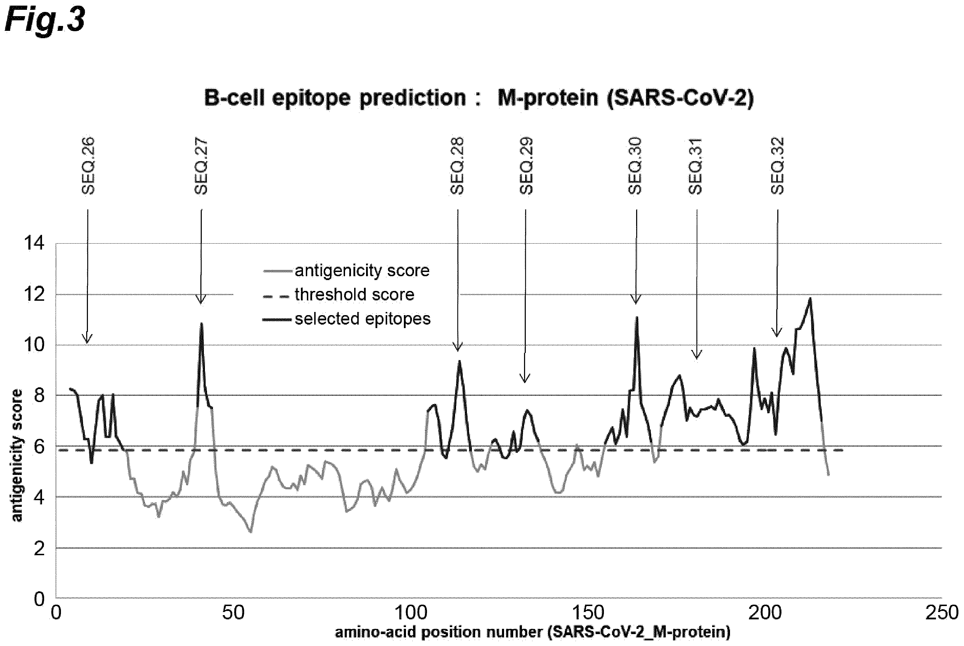

A monoclonal antibody according to the present embodiment that specifically reacts with the M-protein binds to an amino acid region of the M-protein corresponding to amino acid positions from 1 to 24 (SEQ ID NO: 26), from 33 to 50 (SEQ ID NO: 27), from 101 to 120 (SEQ ID NO: 28), from 121 to 139 (SEQ ID NO: 29), from 146 to 171 (SEQ ID NO: 30), from 169 to 195 (SEQ ID NO: 31), or from 193 to 222 (SEQ ID NO: 32). In Figure 3, obtained by scoring the antigen intensity of the whole M-protein molecule, these amino acid regions have high antigenicity scores as compared with a threshold score, and are polypeptides having a sequence consisting of at least 18 amino acids.

The monoclonal antibody according to the present embodiment that specifically reacts with the M-protein preferably binds to an amino acid region of the M-protein corresponding to amino acid positions from 1 to 19, from 38 to 45, from 103 to 118, from 122 to 138, from 155 to 168, from 172 to 195, or from 196 to 216. In Figure 3, obtained by scoring the antigen intensity of the whole M-protein molecule, these amino acid regions have higher antigenicity scores as compared with a threshold score, and are polypeptides having a sequence consisting of at least 8 amino acids.

The monoclonal antibody according to the present embodiment that specifically reacts with the M-protein more preferably binds to an amino acid region of the M-protein corresponding to amino acid positions from 1 to 16, from 40 to 44, from 105 to 117, from 123 to 136, from 160 to 167, from 173 to 179, or from 205 to 215. In Figure 3, obtained by scoring the antigen intensity of the whole M-protein molecule, these amino acid regions have the highest antigenicity scores as compared with a threshold score, and are polypeptides having a sequence consisting of at least 5 amino acids.

The E-protein (envelope protein; hereinafter also called merely "E-protein") of SARS-CoV-2 consists of the sequence of 75 amino acids set forth by SEQ ID NO: 4.

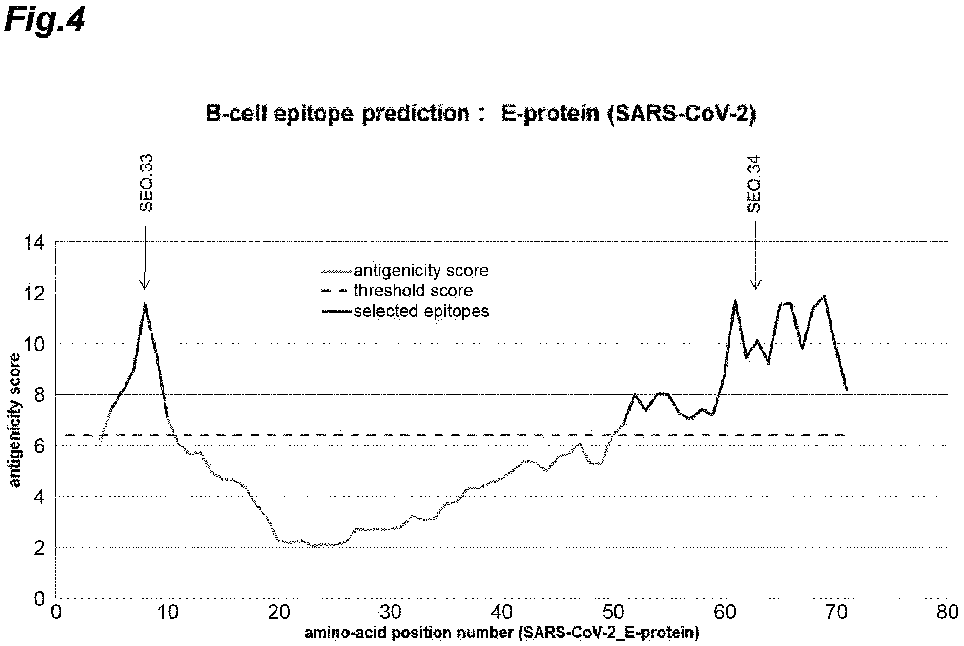

A monoclonal antibody according to the present embodiment that specifically reacts with the E-protein binds to an amino acid region of the E-protein corresponding to amino acid positions from 1 to 19 (SEQ ID NO: 33) or from 50 to 75 (SEQ ID NO: 34). In Figure 4, obtained by scoring the antigen intensity of the whole E-protein molecule, these amino acid regions have high antigenicity scores as compared with a threshold score, and are polypeptides having a sequence consisting of at least 19 amino acids.

The monoclonal antibody according to the present embodiment that specifically reacts with the E-protein preferably binds to an amino acid region of the E-protein corresponding to amino acid positions from 4 to 13 or from 51 to 72. In Figure 4, obtained by scoring the antigen intensity of the whole E-protein molecule, these amino acid regions have higher antigenicity scores as compared with a threshold score, and are polypeptides having a sequence consisting of at least 10 amino acids.

The monoclonal antibody according to the present embodiment that specifically reacts with the E-protein more preferably binds to an amino acid region of the E-protein corresponding to amino acid positions from 5 to 10 or from 60 to 71. In Figure 4, obtained by scoring the antigen intensity of the whole E-protein molecule, these amino acid regions have the highest antigenicity scores as compared with a threshold score, and are polypeptides having a sequence consisting of at least 6 amino acids.

An antibody can also be prepared based on the monoclonal antibody according to the present embodiment, and only an antigen binding site can also be separated and used. That is, fragments such as Fab and Fab', F(ab')₂, a single chain antibody (scFv), and VHH produced by well-known methods and having specific antigen binding (antigen-binding fragments) are included in the scope of the present invention. The class of the monoclonal antibody according to the present embodiment is not limited to IgG, and may be IgY, IgM, Camel Ig, or Ig NAR. An "antibody" used herein means "an antibody or an antigen-binding fragment thereof" except when it is clear from the context that an "antibody" used herein does not mean "an antibody or an antigen-binding fragment thereof".

An animal to be immunized can be immunized with a protein of SARS-CoV-2 or a partial peptide thereof (for example, a polypeptide consisting of a specific amino acid region in the above-mentioned S-protein, N-protein, M-protein, or E-protein), and hybridomas can be produced with cells of the animal to be immunized using a well-known immunological technique; or the monoclonal antibody according to the present embodiment can be produced as a recombinant antibody by gene recombination technology to obtain the monoclonal antibody according to the present embodiment. Although the length of the peptide to be used for immunization is not particularly limited, a peptide having preferably 5 or more amino acids, more preferably 10 or more amino acids, and further preferably 13 or more amino acids can be used as an immunogen.

The protein of SARS-CoV-2 to be used as immunogens can also be obtained from culture virus solution, and can also obtained by incorporating DNA encoding the protein of SARS-CoV-2 into plasmid vectors, introducing this into host cells, and expressing the protein.

The protein of SARS-CoV-2 or the partial peptide thereof to be used as an immunogen is expressed as fused protein with protein as illustrated below, and can be also used as an immunogen after purification or without purification. Glutathione S-transferase (GST), maltose-binding protein (MBP), thioredoxin (TRX), a Nus tag, an S tag, an HSV tag, an FLAG tag, a polyhistidine tag, a Strep tag, a Strep-II tag, a Myc tag, an HA tag, a V5 tag, an E tag, a T7 tag, a VSV-G tag, a Glu-Glu tag, an Avi tag, and the like that those skilled in the art use as "protein expression and purification tags" commonly can be used for producing fused protein. It is preferable to cleave the fused protein with these into the protein of SARS-CoV-2 or the partial peptide portion thereof and the other tag portion using a digestive enzyme for separation and purification and then use the protein of SARS-CoV-2 or the partial peptide portion as an immunogen.

The monoclonal antibody can be easily prepared from an immunized animal by a well-known method of Kohler et al. (Kohler et al., Nature, vol. 256, p495-497 (1975)). That is, antibody-producing cells such as splenic cells and lymphocytes are collected from an immunized animal, these are fused with mouse myeloma cells by a usual method to produce hybridomas, the obtained hybridomas are cloned by limiting dilution or the like, and monoclonal antibodies that cause antigen-antibody reaction with the proteins of SARS-CoV-2 are selected among monoclonal antibodies produced by the cloned hybridomas.

A well-known immunoglobulin purification method can be used for purifying the monoclonal antibodies from ascites or a culture supernatant. Examples include fractionation by salt precipitation using ammonium sulfate or sodium sulfate, PEG fractionation, ethanol fractionation, DEAE ion exchange chromatography, and gel filtration. The purification is feasible depending on the classes of an immune animal species and the monoclonal antibody also by affinity chromatography using a carrier bound to any of protein A, protein G, and protein L.

A method for detecting SARS-CoV-2 according to the present embodiment includes contacting a monoclonal antibody or an antigen-binding fragment thereof that specifically reacts with a protein of SARS-CoV-2, namely at least one protein selected from the group consisting of S-protein, N-protein, M-protein, and E-protein, with a specimen and detecting SARS-CoV-2 by an immunological measuring method.

Examples of the specimen to be used in the detecting method according to the present embodiment include biological samples such as body fluids such as blood, serum, plasma, urine, semen, cerebrospinal fluid, saliva, sweat, tears, ascites, or amniotic fluid; mucus; feces; blood vessels or internal organs such as the liver; tissue; cells or extract thereof of humans or animals in which the proteins of SARS-CoV-2 may be contained, and the specimen is preferably cells or secreta from the oral cavity, the tonsils, the nasal cavity, the pharynx, the larynx, the trachea, the bronchus, or the lungs, or the like; nasal swab; pharynx swab; gargled liquid; expectoration; tracheal aspirate fluid; bronchoalveolar lavage fluid; or the like that is easily collected. Methods for collecting these specimens are not limited, and well-known methods can be adopted. Specific examples include a method using a swab.

If methods for measuring proteins of SARS-CoV-2 according to the present embodiment are immunological measuring methods well-known to those skilled in the art, all the methods can be adopted. Examples of the immunological measuring methods include competitive methods, coagulating methods, western blotting, immunostaining, and sandwich methods.

As a method for measuring a protein of SARS-CoV-2 according to the present embodiment, a sandwich method using at least two monoclonal antibodies is preferable. The sandwich method itself is well-known in the field of immunoassay, and can be performed, for example, by immunochromatography, ELISA, or the like. The measuring method according to the present embodiment can be performed by a well-known sandwich method except that monoclonal antibodies specifically reactive to the above-mentioned protein of SARS-CoV-2 are used.

If the protein of SARS-CoV-2 to be measured in the sandwich method is S-protein, it is preferable to use at least two monoclonal antibodies that can simultaneously bind to amino acid regions selected from the group consisting of amino acid regions corresponding to amino acid positions from 66 to 85 (SEQ ID NO: 5), from 87 to 107 (SEQ ID NO: 6), from 105 to 131 (SEQ ID NO: 7), from 140 to 157 (SEQ ID NO: 8), from 155 to 170 (SEQ ID NO: 9), from 522 to 541 (SEQ ID NO: 10), from 538 to 562 (SEQ ID NO: 11), from 595 to 611 (SEQ ID NO: 12), from 670 to 693 (SEQ ID NO: 13), from 761 to 797 (SEQ ID NO: 14), from 802 to 818 (SEQ ID NO: 15), and from 1235 to 1273 (SEQ ID NO: 16).

If the protein of SARS-CoV-2 to be measured in the sandwich method is N-protein, it is preferable to use at least two monoclonal antibodies that can simultaneously bind to amino acid regions selected from the group consisting of amino acid regions corresponding to amino acid positions from 1 to 18 (SEQ ID NO: 17), from 19 to 49 (SEQ ID NO: 18), from 174 to 207 (SEQ ID NO: 19), from 230 to 252 (SEQ ID NO: 20), from 247 to 267 (SEQ ID NO: 21), from 336 to 350 (SEQ ID NO: 22), from 362 to 379 (SEQ ID NO: 23), from 377 to 395 (SEQ ID NO: 24), and from 401 to 419 (SEQ ID NO: 25).

If the protein of SARS-CoV-2 to be measured in the sandwich method is M-protein, it is preferable to use at least two monoclonal antibodies that can simultaneously bind to amino acid regions selected from the group consisting of amino acid regions corresponding to amino acid positions from 1 to 24 (SEQ ID NO: 26), from 33 to 50 (SEQ ID NO: 27), from 101 to 120 (SEQ ID NO: 28), from 121 to 139 (SEQ ID NO: 29), from 146 to 171 (SEQ ID NO: 30), from 169 to 195 (SEQ ID NO: 31), and from 193 to 222 (SEQ ID NO: 32).

If the protein of SARS-CoV-2 to be measured in the sandwich method is E-protein, it is preferable to use two monoclonal antibodies that can simultaneously bind to amino acid regions corresponding to amino acid positions from 1 to 19 (SEQ ID NO: 33) and from 50 to 75 (SEQ ID NO: 34).

A detection kit for SARS-CoV-2 according to the present embodiment comprises a monoclonal antibody or an antigen-binding fragment thereof that specifically reacts with at least one protein selected from the group consisting of proteins of SARS-CoV-2, namely S-protein, N-protein, M-protein, and E-protein.

If the detection kit for SARS-CoV-2 according to the present embodiment is in a form used for the above-mentioned immunological measuring methods, any detection kit can be adopted, and the detection kit is however preferably in the form of immunochromatographic strip, in which the protein of SARS-CoV-2 can be measured accurately, rapidly, and easily.

One preferable aspect of the detection kit for SARS-CoV-2 according to the present embodiment comprises at least two monoclonal antibodies or antigen-binding fragments thereof that can simultaneously bind to amino acid regions of the S-protein selected from the group consisting of amino acid regions corresponding to amino acid positions from 66 to 85 (SEQ ID NO: 5), from 87 to 107 (SEQ ID NO: 6), from 105 to 131 (SEQ ID NO: 7), from 140 to 157 (SEQ ID NO: 8), from 155 to 170 (SEQ ID NO: 9), from 522 to 541 (SEQ ID NO: 10), from 538 to 562 (SEQ ID NO: 11), from 595 to 611 (SEQ ID NO: 12), from 670 to 693 (SEQ ID NO: 13), from 761 to 797 (SEQ ID NO: 14), from 802 to 818 (SEQ ID NO: 15), and from 1235 to 1273 (SEQ ID NO: 16).

One preferable aspect of the detection kit for SARS-CoV-2 according to the present embodiment comprises at least two monoclonal antibodies or antigen-binding fragments thereof that can simultaneously bind to amino acid regions of the N-protein selected from the group consisting of amino acid regions corresponding to amino acid positions from 1 to 18 (SEQ ID NO: 17), from 19 to 49 (SEQ ID NO: 18), from 174 to 207 (SEQ ID NO: 19), from 230 to 252 (SEQ ID NO: 20), from 247 to 267 (SEQ ID NO: 21), from 336 to 350 (SEQ ID NO: 22), from 362 to 379 (SEQ ID NO: 23), from 377 to 395 (SEQ ID NO: 24), and from 401 to 419 (SEQ ID NO: 25).

One preferable aspect of the detection kit for SARS-CoV-2 according to the present embodiment comprises at least two monoclonal antibodies or antigen-binding fragments thereof according to claim 8 that can simultaneously bind to amino acid regions of the M-protein selected from the group consisting of amino acid regions corresponding to amino acid positions from 1 to 24 (SEQ ID NO: 26), from 33 to 50 (SEQ ID NO: 27), from 101 to 120 (SEQ ID NO: 28), from 121 to 139 (SEQ ID NO: 29), from 146 to 171 (SEQ ID NO: 30), from 169 to 195 (SEQ ID NO: 31), and from 193 to 222 (SEQ ID NO: 32).

One preferable aspect of the detection kit for SARS-CoV-2 according to the present embodiment comprises two monoclonal antibodies or antigen-binding fragments thereof that can simultaneously bind to amino acid regions of the E-protein corresponding to amino acid positions from 1 to 19 (SEQ ID NO: 33) and from 50 to 75 (SEQ ID NO: 34).

[Antibody against SARS-CoV-2 and human infectious coronavirus other than SARS-CoV-2; method for detecting SARS-CoV-2 and human infectious coronavirus other than SARS-CoV-2 using the antibody; and detection kit comprising the antibody]

The monoclonal antibody according to the present embodiment specifically reacts with a structural protein of SARS-CoV-2, namely at least one protein selected from the group consisting of S-protein (surface protein), N-protein (nucleocapsid protein), M-protein (membrane protein), and E-protein (envelope protein) of SARS-CoV-2 and a structural protein of a human infectious coronavirus other than SARS-CoV-2, namely at least one protein selected from the group consisting of S-protein (surface protein), N-protein (nucleocapsid protein), M-protein (membrane protein), and E-protein (envelope protein) of a human infectious coronavirus other than SARS-CoV-2.

"Specifically" in the present embodiment means that, in a liquid system in which protein and the monoclonal antibody according to the present embodiment are mixed, the antibody does not cause antigen-antibody reaction with protein components other than the protein of SARS-CoV-2 and the protein of the human infectious coronavirus other than SARS-CoV-2 at a detectable level, or even though the antibody causes some binding or association reaction, the antibody causes only reaction clearly weaker than an antigen-antibody reaction of the antibody with the protein of SARS-CoV-2 and the protein of the human infectious coronavirus other than SARS-CoV-2.

If the human infectious coronavirus other than SARS-CoV-2 is a coronavirus that causes critical respiratory symptoms in humans, the human infectious coronavirus other than SARS-CoV-2 is not particularly limited, and is however preferably MERS-CoV and/or SARS-CoV, and is more preferably SARS-CoV.

The monoclonal antibody according to the present embodiment can be prepared in the method described in the above-mentioned [Antibody against SARS-CoV-2, method for detecting SARS-CoV-2 using the antibody, and detection kit comprising the antibody] in the same way.

A method for detecting SARS-CoV-2 and a human infectious coronavirus other than SARS-CoV-2 according to the present embodiment includes contacting a monoclonal antibody or an antigen-binding fragment thereof that specifically reacts with a structural protein of SARS-CoV-2, namely at least one protein selected from the group consisting of S-protein, N-protein, M-protein, and E-protein of SARS-CoV-2, and a structural protein of a human infectious coronavirus other than SARS-CoV-2, namely at least one protein selected from the group consisting of S-protein, N-protein, M-protein, and E-protein of the human infectious coronavirus other than SARS-CoV-2, with a specimen and detecting SARS-CoV-2 and the human infectious coronavirus other than SARS-CoV-2 by an immunological measuring method.

The specimen to be used in the method according to the present embodiment and the measurement of the protein of SARS-CoV-2 and the protein of the human infectious coronavirus other than SARS-CoV-2 are the same as the specimen and the measuring method described in the above-mentioned [Antibody against SARS-CoV-2, method for detecting SARS-CoV-2 using the antibody, and detection kit comprising the antibody].

A detection kit for SARS-CoV-2 and a human infectious coronavirus other than SARS-CoV-2 according to the present embodiment comprises a monoclonal antibody or an antigen-binding fragment thereof that specifically reacts with a structural protein of SARS-CoV-2, namely at least one protein selected from the group consisting of S-protein, N-protein, M-protein, and E-protein of SARS-CoV-2, and a structural protein of a human infectious coronavirus other than SARS-CoV-2, namely at least one protein selected from the group consisting of S-protein, N-protein, M-protein, and E-protein of the human infectious coronavirus other than SARS-CoV-2.

### [Epitope (polypeptide) that antibody for detecting SARS-CoV-2 recognizes]

A polypeptide consisting of any one amino acid sequence of SEQ ID NOS: 5 to 34 according to the present embodiment can be used as an epitope that an antibody for detecting SARS-CoV-2 present in a specimen recognizes. The polypeptide according to the present embodiment can be used as an epitope that the antibody for detecting a human infectious coronavirus other than SARS-CoV-2 in addition to SARS-CoV-2 that is present in a specimen recognizes.

The polypeptide according to the present embodiment can be used as a hapten for detecting the antibody against SARS-CoV-2 present in the specimen (preferably in blood) (namely, anti-SARS-CoV-2 antibody titer produced by the defense mechanism against SARS-CoV-2 in the living body). As one embodiment of the present invention, a method for detecting an anti-SARS-CoV-2 antibody in a specimen including contacting one or two or more polypeptides having amino acid sequences of SEQ ID NOS: 5 to 34 with a specimen (preferably blood) and detecting an anti-SARS-CoV-2 antibody by an immunological measuring method is accordingly provided. As one embodiment of the present invention, a detection kit for an anti-SARS-CoV-2 antibody comprising one or two or more polypeptides having amino acid sequences of SEQ ID NOS: 5 to 34 is provided.

The polypeptide according to the present embodiment can be used as a hapten for detecting the antibody against SARS-CoV-2 and the antibody against the human infectious coronavirus other than SARS-CoV-2 (namely, an anti-human infectious coronavirus antibody titer produced by the defense mechanism against the human infectious coronavirus other than SARS-CoV-2 in the living body) present in a specimen (preferably in blood). As one embodiment of the present invention, a method for detecting an anti-SARS-CoV-2 antibody and an anti-human infectious coronavirus other than the anti-SARS-CoV-2 antibody in a specimen including contacting one or two or more polypeptides having an amino acid sequence of SEQ ID NOS: 5 to 34 with a specimen (preferably blood) and detecting an anti-SARS-CoV-2 antibody and an anti-human infectious coronavirus antibody other than the anti-SARS-CoV-2 antibody by an immunological measuring method is accordingly provided. As one embodiment of the present invention, a detection kit for an anti-SARS-CoV-2 antibody and an anti-human infectious coronavirus antibody other than the anti-SARS-CoV-2 antibody comprising one or two or more polypeptides having amino acid sequences of SEQ ID NOS: 5 to 34 is provided.

A polypeptide according to the present embodiment has availability as a basic skeleton of a vaccine for preventing infection with SARS-CoV-2 (for example, peptide vaccine, cocktail peptide vaccine, fused peptides, fragment domains comprising the peptides). The polypeptide according to the present embodiment has availability as a basic skeleton of a vaccine for preventing infection with a human infectious coronavirus other than SARS-CoV-2 in addition to SARS-CoV-2.

The polypeptide according to the present embodiment has availability as a basic skeleton of a vaccine for preventing a SARS-CoV-2 infection from becoming severe (for example, peptide vaccine, cocktail peptide vaccine, fused peptides, fragment domains comprising the peptides). The polypeptide according to the present embodiment has availability as a basic skeleton of a vaccine for preventing an infection due to a human infectious coronavirus other than SARS-CoV-2 in addition to a SARS-CoV-2 infection from becoming severe.

The monoclonal antibody or an antigen-binding fragment thereof that specifically reacts with the polypeptide according to the present embodiment has availability as an antibody drug (neutralizing antibody) for treating a SARS-CoV-2 infection. The monoclonal antibody or the antigen-binding fragment thereof that specifically reacts with the polypeptide according to the present embodiment has availability as an antibody drug (neutralizing antibody) for treating an infection due to a human infectious coronavirus other than SARS-CoV-2 in addition to a SARS-CoV-2 infection.

As long as the above-mentioned human infectious coronavirus other than SARS-CoV-2 in the embodiments is a coronavirus that causes critical respiratory symptoms in humans, the human infectious coronavirus is not particularly limited, and the human infectious coronavirus is preferably MERS-CoV and/or SARS-CoV, and more preferably SARS-CoV.

### Examples

### [Example 1: Measurement of antibody titer]

Peptide conjugates in which a total of 24 polypeptides having a sequence of SEQ ID NOS: 5, 6, 7, 8, 9, 10, 11, 13, 15, 16, 18, 19, 20, 21, 23, 24, 25, 26, 27, 30, 31, 32, 33, or 34 are covalently bound to Blue Carrier Protein (mollusk-derived hemocyanin) and BSA (bovine serum albumin) individually and separately based on the MBS method (Syed Salman Lateef, et al. J Biomol Tech. 2007 Jul; 18(3): 173-176.) were prepared. The peptide conjugates using Blue Carrier Protein were each intraabdominally administered to three BALB/c mice in a dose of 100 µg/200 µL/head as immunogens. A Sigma Adjuvant System was used as an adjuvant. Blood was collected from the tail vein over time during the immune period. The antibody titers were confirmed in the following procedure by the peptide conjugates using BSA solid phased ELISA (enzyme-linked immunosorbent assay).
1) A total of 24 BSA peptide conjugate solutions diluted with PBS to 2 µg/mL were each added to a 96-well immunoplate in an amount of 100 µL/well and immobilized at 4°C overnight.
2) The BSA peptide conjugate solutions were discarded, washing was performed with a wash buffer (0.05% Tween20/PBS) twice, Blocking One (produced by NACALAI TESQUE, INC.) diluted with MilliQ water 5 times was added in an amount of 250 µL/well and incubation was performed at 25°C for around 1 hour for blocking.
3) The blocking solution was discarded, washing was performed with the wash buffer twice, mouse plasma diluted with the wash buffer 100 to 100,000 times was then added in an amount of 100 µL/well and incubation was performed at 25°C for 2 hours.
4) The plasma solution was discarded, washing was performed with the wash buffer 3 times, Peroxidase-Conjugated Goat Anti-Mouse Immunoglobulins (produced by Agilent Technologies, Inc.) diluted with the wash buffer 5,000 times was then added in an amount of 100 µL/well and incubation was performed at room temperature for 1 hour.
5) The antibody solution was discarded, washing was performed with the wash buffer 3 times, and TMB solution (TMB One-Step Substrate System: produced by Agilent Technologies, Inc.) was then added in an amount of 100 µL/well.
6) Incubation was performed at 25°C for 20 minutes, and 2N H₂SO₄ was then added in an amount of 100 µL/well to stop the reaction.
7) The absorbances at 450 nm/630 nm were measured with a plate reader.

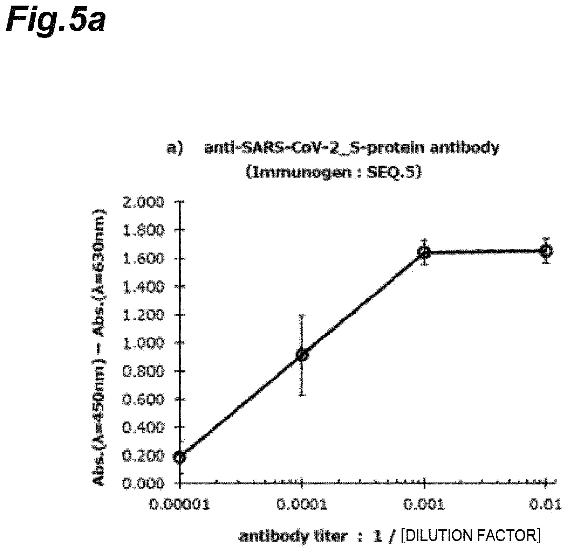

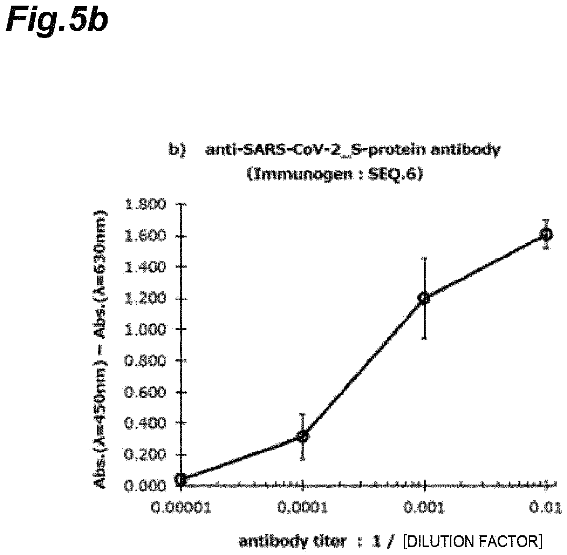

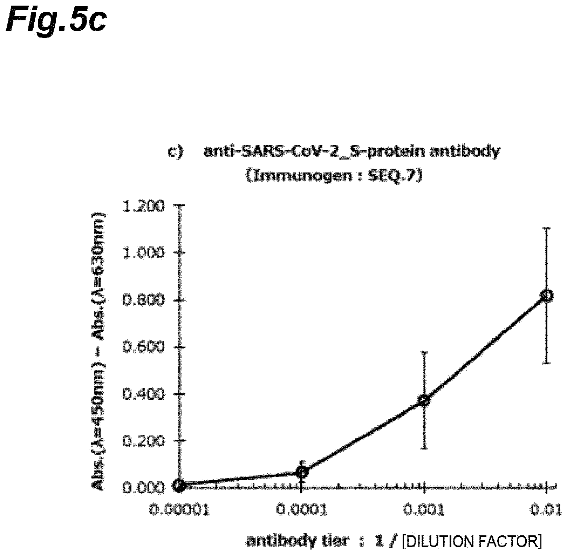

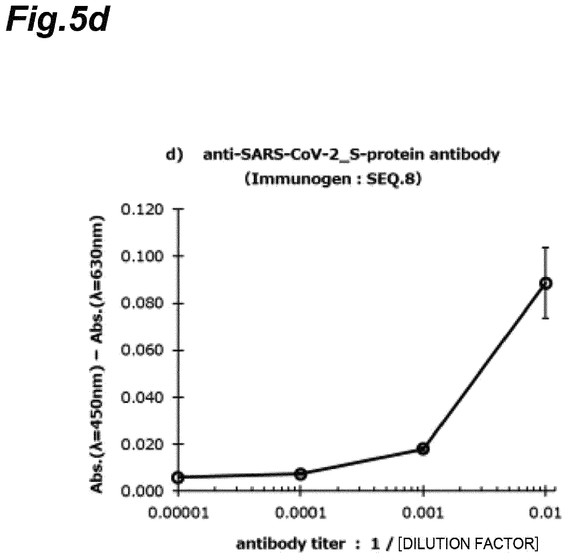

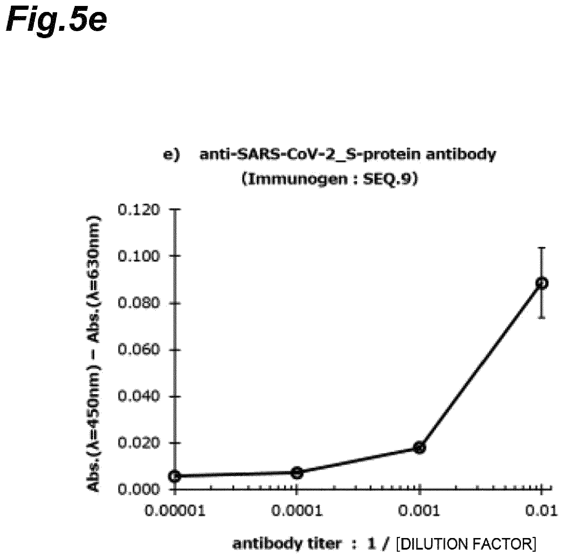

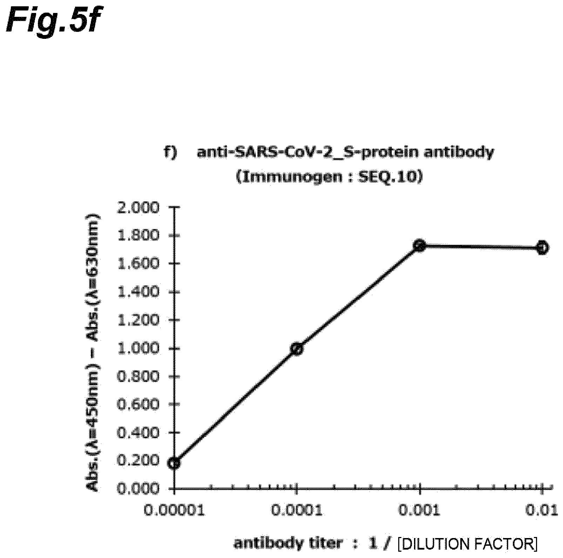

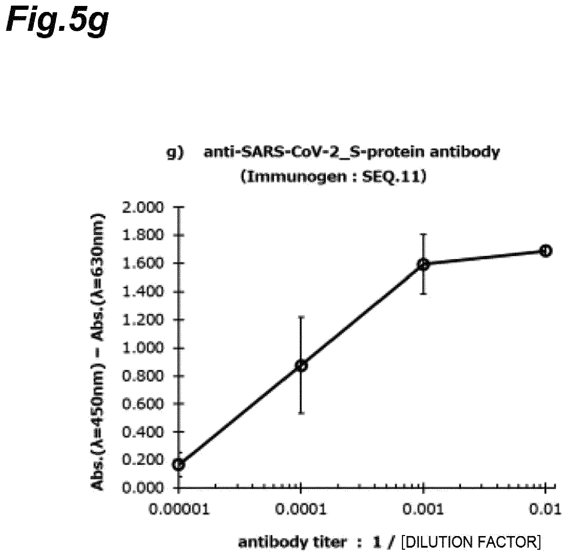

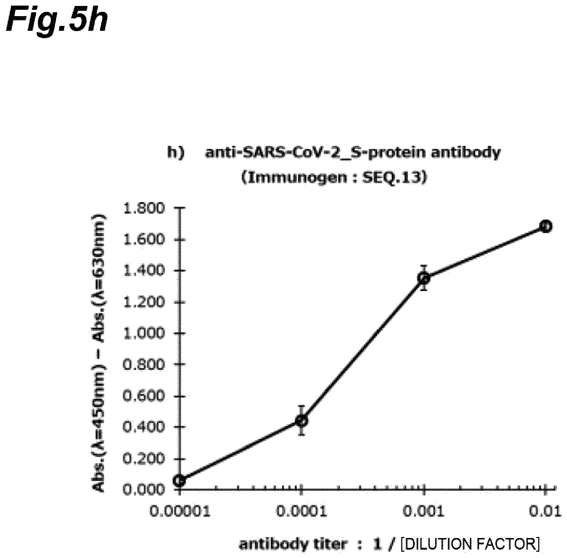

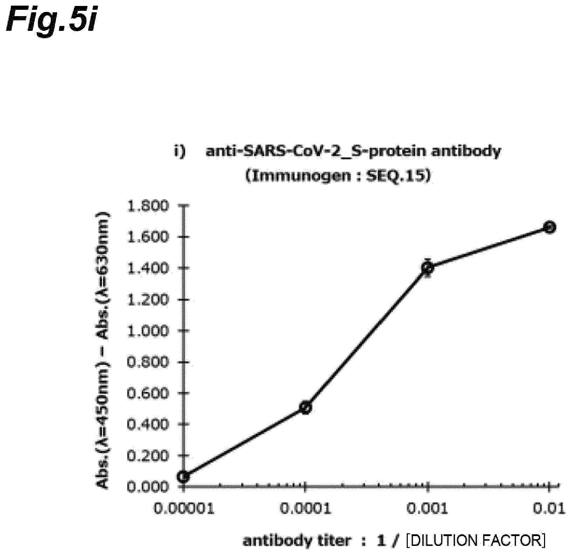

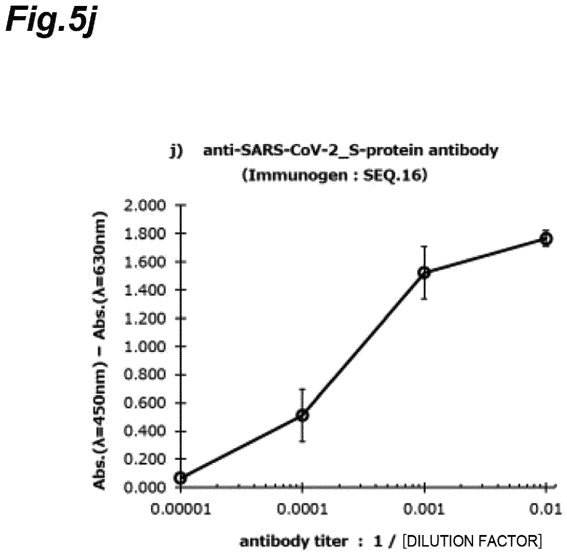

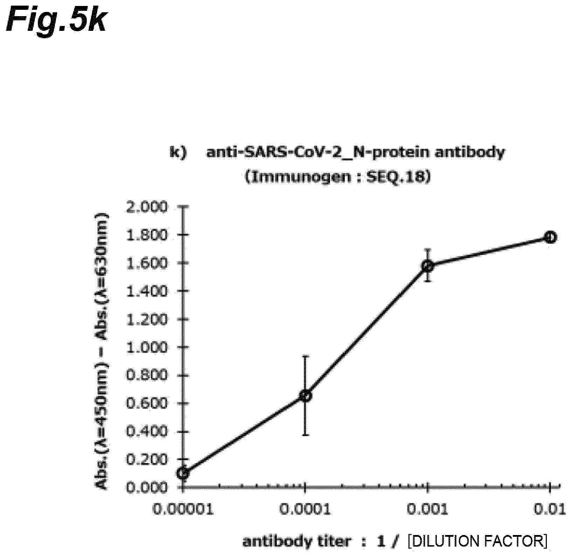

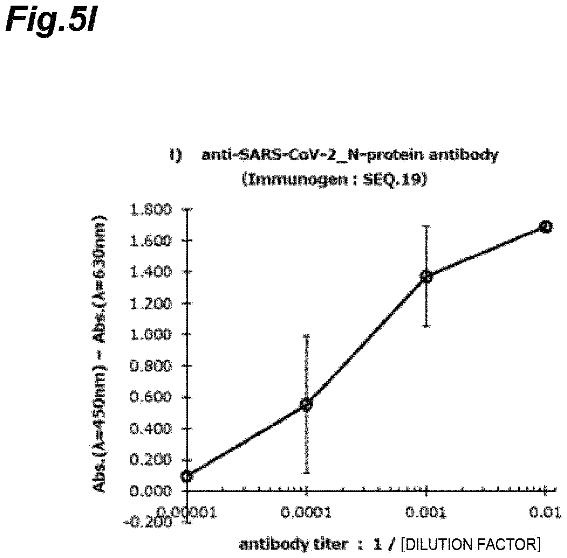

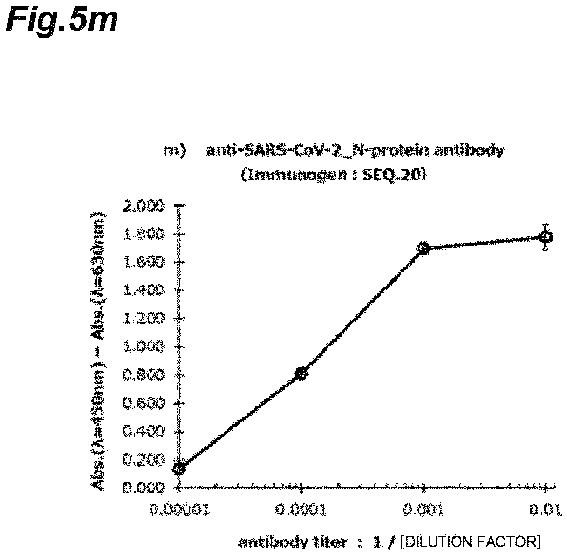

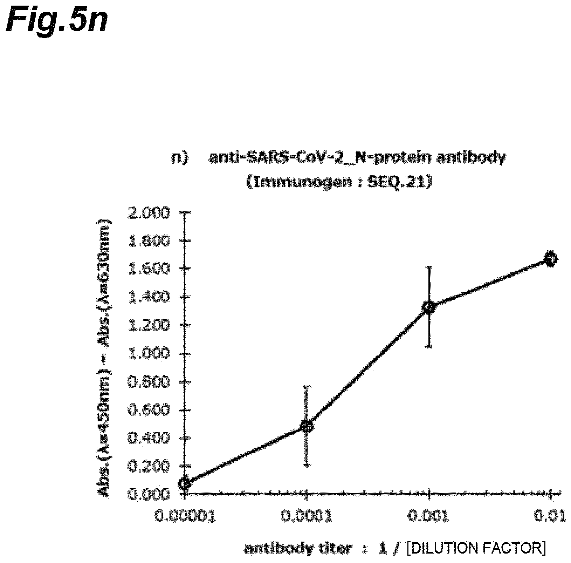

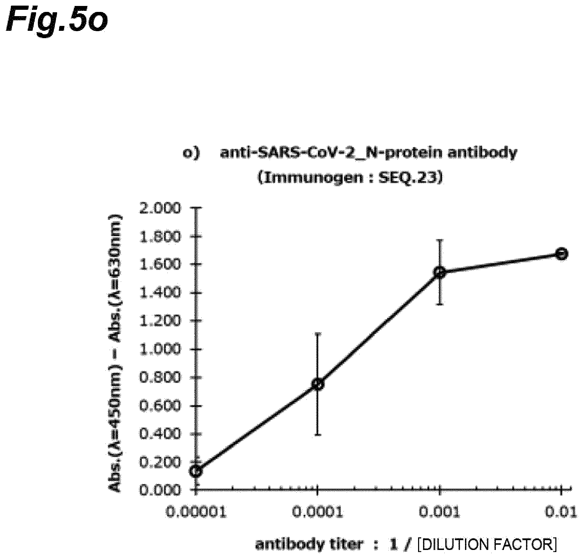

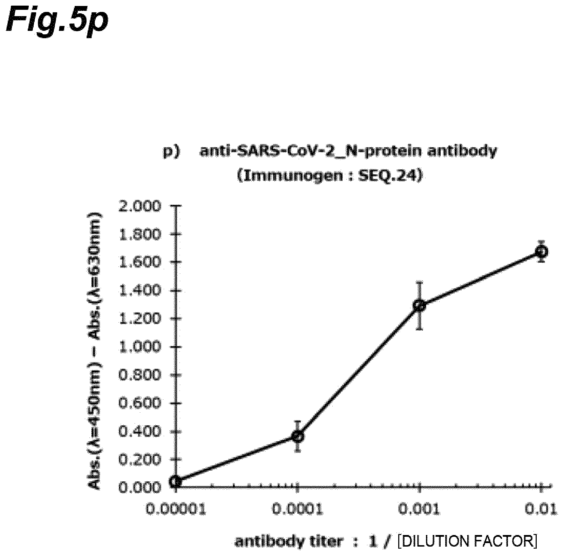

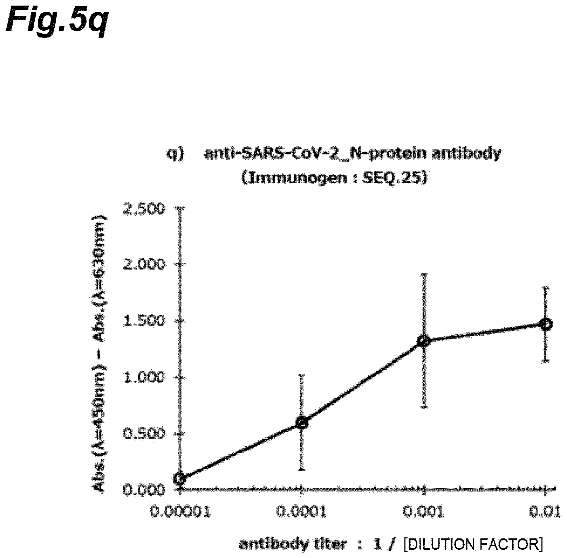

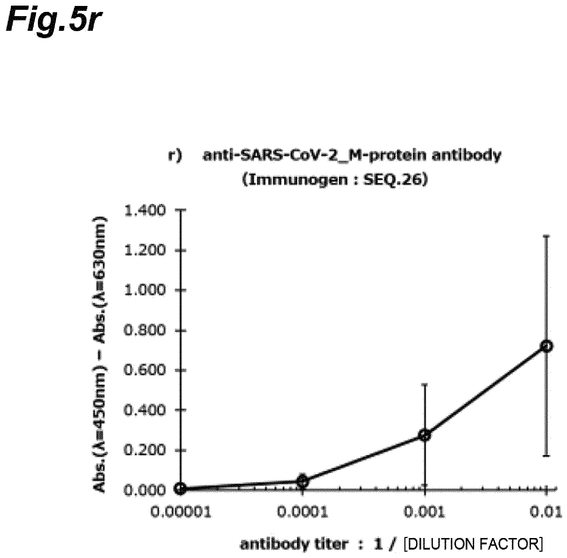

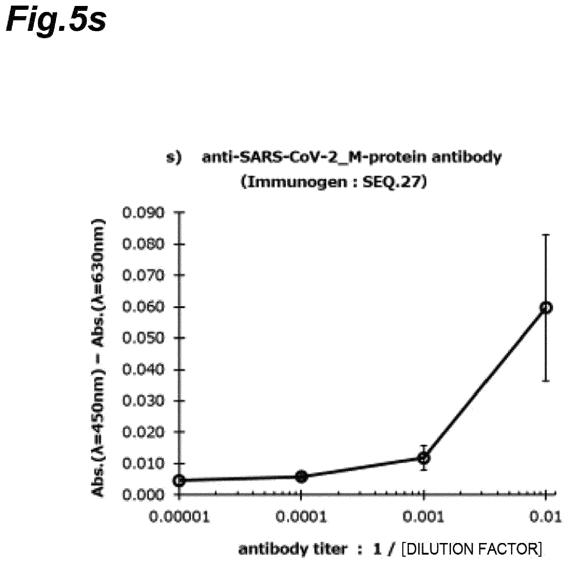

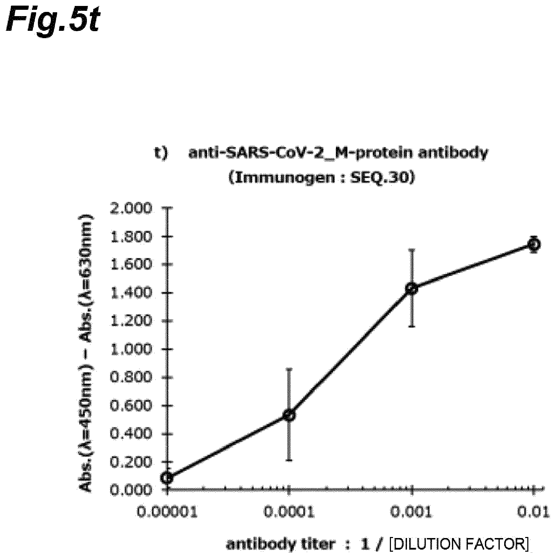

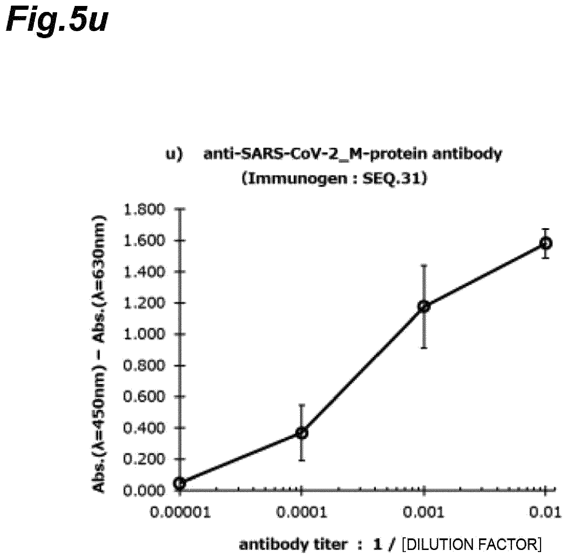

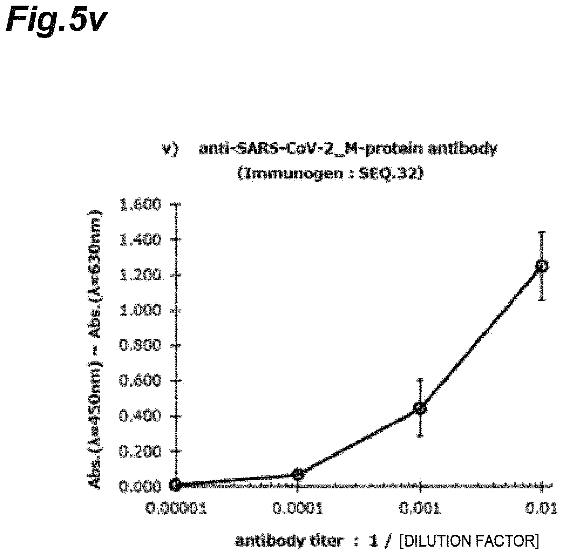

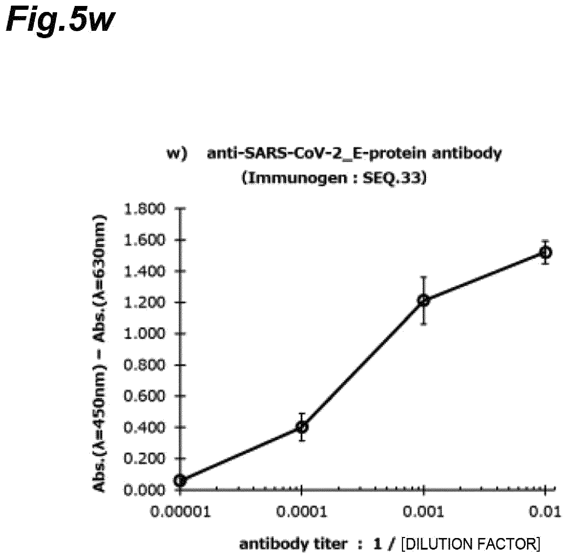

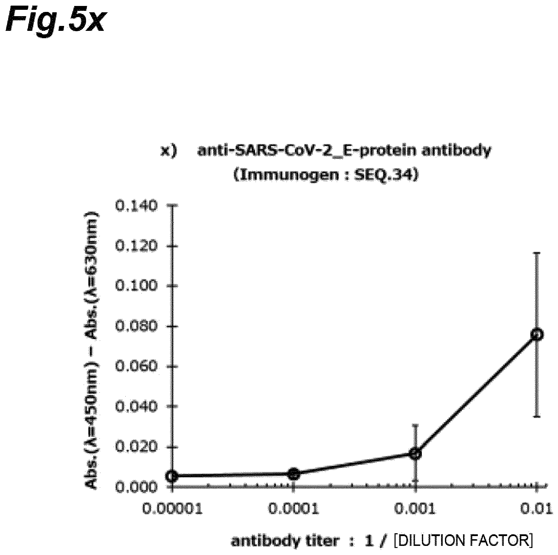

The results are shown in Figures 5a to 5x. The error bars (SD) in the figures are the differences between immune mouse individuals (n = 3).

As shown in Figures 5a to 5x, it was confirmed that the antibody titers against the epitope peptides of S-protein, N-protein, M-protein, and E-protein increased.

### [Example 2: Reactivity of antibody with SARS-CoV-2 inactivated antigen]

The reactivities of the total of 24 antibodies obtained in Example 1 with a SARS-CoV-2 inactivated antigen were confirmed in the following procedure by ELISA (enzyme-linked immunosorbent assay) using a microtiter plate on which the inactivated antigen of SARS-CoV-2 (EPI ISL406034) was immobilized.
1) 24 antibodies in total obtained in Example 1 were each added to a 96-well immunoplate on which a SARS-CoV-2 inactivated antigen was immobilized in an amount of 100 µL/well and incubation was performed at 37°C for 1 hour.
2) The plasma solution was discarded, washing was performed with a wash buffer (0.05% Tween20/PBS) twice, Peroxidase-conjugated AffiniPure Goat Anti-Mouse IgG (subclasses 1 + 2a + 2b + 3) and Fey Fragment Specific (produced by Jackson ImmunoResearch Inc.) diluted with a dilution buffer (0.05% Tween20, 0.5% BSA/PBS) 25,000 times were added in an amount of 100 µL/well and incubation was performed at 37°C for 1 hour.
3) The antibody solution was discarded, washing was performed with the wash buffer twice, and TMB solution (TMB One-Step Substrate System: produced by Agilent Technologies, Inc.) was added in an amount of 100 µL/well.
4) Incubation was performed at 25°C for 20 minutes, and 2N H₂SO₄ was then added in an amount of 100 µL/well to stop the reaction.
5) The absorbances of 450 nm/630 nm were measured with a plate reader.

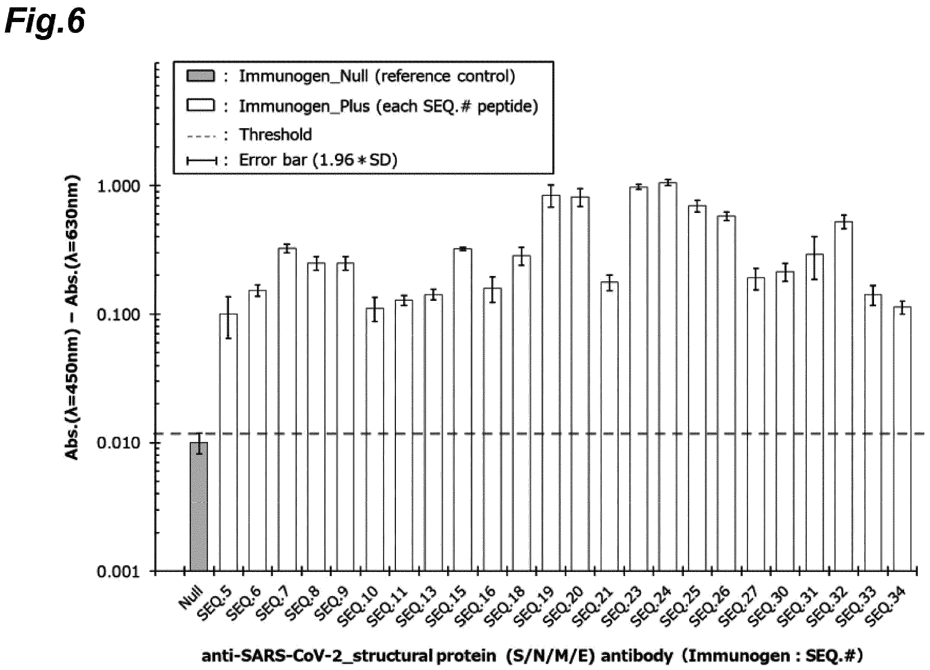

The results are shown in Figure 6. The error bars (1.96 × SD) in the figure are the differences between measurements (n = 3).

As shown in Figure 6, it was confirmed that the total of 24 antibodies obtained in Example 1 all react with the SARS-CoV-2 inactivated antigen.

[Example 3: Analysis of epitope of antibody present in antiserum when SARS-CoV-2 inactivated antigen was used as immunogen]

BALB/c mice were immunized in the same procedure as in Example 1 using the SARS-CoV-2 inactivated antigen used in Example 2 as an immunogen. The epitopes of the antibody present in the obtained antiserum were analyzed in the following procedure.
1) A SARS-CoV-2 N-protein peptide set containing the following polypeptides (BioTides(TM) Biotinylated Peptides: produced by JPT Peptide Technologies GmbH) was used, and the polypeptides were obtained by continuously sliding one amino acid along the N-protein sequence of SARS-CoV-2 (SEQ ID NO: 2) from the N-terminus to the C-terminus side to obtain polypeptides consisting of 15 contiguous amino acids, labeling the N-termini of the polypeptide chains with biotin, and adding one glycine residue to the C-terminus of each of the polypeptide chains.
2) The BioTides(TM) Biotinylated Peptides, described in the above-mentioned 1), was dissolved in DMF (N,N-dimethylformamide), BioTides(TM) Biotinylated Peptides-immobilized LumAvidin Microspheres were prepared through the biotin-avidin reaction with LumAvidin Microspheres (produced by Luminex Corporation). Details on this preparation were in accordance with the xMAP(R) Cookbook (Chapter 4.2.1) of Luminex Corporation.
3) 50 µL of a solution obtained by immunizing a BALB/c mouse to a SARS-CoV-2 inactivated antigen to obtain antiserum and diluting the antiserum with PBS-TBN (0.02% Tween20, 0.1% BSA/PBS) 100 times and 50 µL of a beads mix in which the BioTides(TM) Biotinylated Peptides-immobilized LumAvidin Microspheres prepared in the above-mentioned 2) were mixed with 500 beads of LumAvidin Microspheres with respect to each of the N-protein peptides of SARS-CoV-2 were mixed, and the mixture was then incubated at 37°C for 1 hour.
4) After centrifugation at 3000 rpm for 5 minutes, the supernatant of the reaction solution was discarded, and washing was performed with a wash buffer (0.02% Tween20/PBS) twice.
5) 100 µL of a solution obtained by diluting R-Phycoerythrin AffiniPure Goat Anti-Mouse IgG (subclasses 1 + 2a + 2b + 3) and Fey Fragment Specific (produced by Jackson ImmunoResearch Inc.) with PBS-TBN 200 times was added to the beads mix after the reaction in the above-mentioned 4) and incubation was performed at 37°C for 1 hour.
6) After centrifugation at 3000 rpm for 5 minutes, the supernatant of the reaction solution was discarded, and washing was performed with the wash buffer twice.
7) 50 µL PBS-BN (1% BSA/PBS) was added to the beads mix after the reaction in the above-mentioned 6) to prepare a beads suspension.
8) The beads suspension prepared in the above-mentioned 7) was measured for the MFI (median fluorescence intensity) using a Bio-Plex 200 (manufactured by Bio-Rad Laboratories, Inc.). As Blank (reference control), measurement was also performed under the condition that only PBS-TBN that was a diluent was used for a measurement sample instead of the antiserum in the above-mentioned 3), and a value obtained by deducting the measured value of Blank from the measured value of the antiserum was expressed as ΔMFI (-Blank).

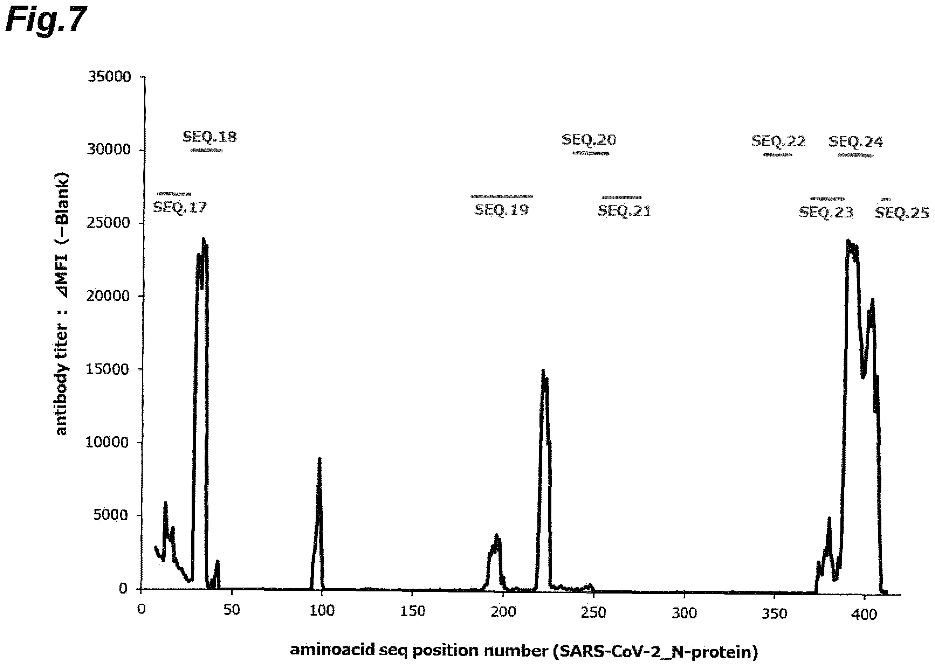

The results are shown in Figure 7.

As shown in Figure 7, it was confirmed that the antiserum obtained in Example 3 had dominant antibody titers against N-protein epitope sequences other than SEQ ID NOS: 21 and 22.

### [Example 4: Analysis of epitopes of antibody present in antiserum when recombinant N-protein was used as immunogen]

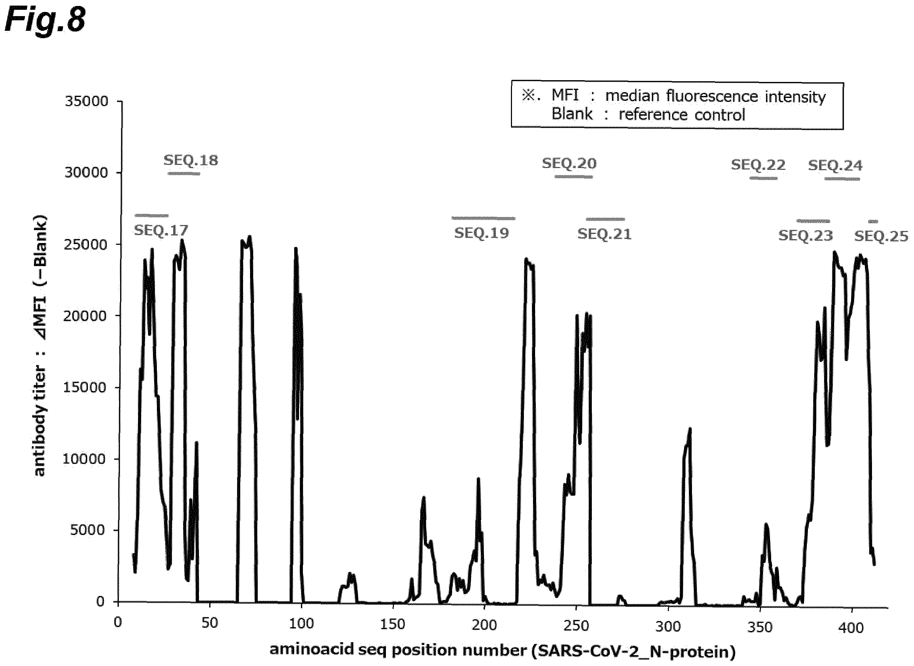

BALB/c mice were immunized in the same procedure as in Example 1 using recombinant N-protein (full-length; SEQ ID NO: 2) as an immunogen. The epitopes of an antibody present in the obtained antiserum were analyzed in the same procedure as in Example 3. The results are shown in Figure 8.

As shown in Figure 8, it was confirmed that the antiserum obtained in Example 4 had dominant antibody titers against all the epitope sequences of the N-protein having SEQ ID NOS: 17 to 25.

### [Example 5: Reactivities of antibody in antiserum with epitope sequences of N-protein]

The reactivities of the antibody in the above-mentioned antiserum with the epitope sequences of the N-protein having SEQ ID NOS: 17 to 25 were confirmed in the following procedure using the antiserums obtained in Example 3 and Example 4.
1) The N-termini of the polypeptides having SEQ ID NOS: 17 to 25 were labeled with biotin, the polypeptides were dissolved in DMF (N,N-dimethylformamide), biotinylated epitope peptides-immobilized LumAvidin Microspheres were prepared through the biotin-avidin reaction with LumAvidin Microspheres (produced by Luminex Corporation). Details on this preparation were in accordance with xMAP(R) Cookbook (Chapter 4.2.1) of Luminex Corporation.
2) 50 µL of a solution obtained by diluting the antiserum obtained in each of Example 3 and Example 4 with PBS-TBN (0.02% Tween20, 0.1% BSA/PBS) 100 times and 50 µL of a beads mix in which the biotinylated epitope peptides-immobilized LumAvidin Microspheres prepared above were mixed with 500 beads of LumAvidin Microspheres with respect to each of the polypeptides having SEQ ID NOS: 17 to 25 were mixed, and the mixture was then incubated at 37°C for 1 hour.
3) After centrifugation at 3000 rpm for 5 minutes, the supernatant of the reaction solution was discarded, and washing was performed with a wash buffer (0.02% Tween20/PBS) twice.
4) 100 µL of a solution obtained by diluting R-Phycoerythrin AffiniPure Goat Anti-Mouse IgG (subclasses 1 + 2a + 2b + 3) and Fcγ Fragment Specific (produced by Jackson ImmunoResearch Inc.) with PBS-TBN 200 times was added to the beads mix after the reaction in the above-mentioned 3) and incubation was performed at 37°C for 1 hour.
5) After centrifugation at 3000 rpm for 5 minutes, the supernatant of the reaction solution was discarded, and washing was performed with the wash buffer twice.
6) 50 µL PBS-BN (1% BSA/PBS) was added to the beads mix after the reaction in the above-mentioned 5) to prepare a beads suspension.
7) The beads suspension prepared in the above-mentioned 6) was measured for the MFI (median fluorescence intensity) using a Bio-Plex 200 (manufactured by Bio-Rad Laboratories, Inc.). As Blank (reference control), measurement was also performed under the condition that only PBS-TBN that was a diluent was used for a measurement sample instead of the antiserum in the above-mentioned 2).

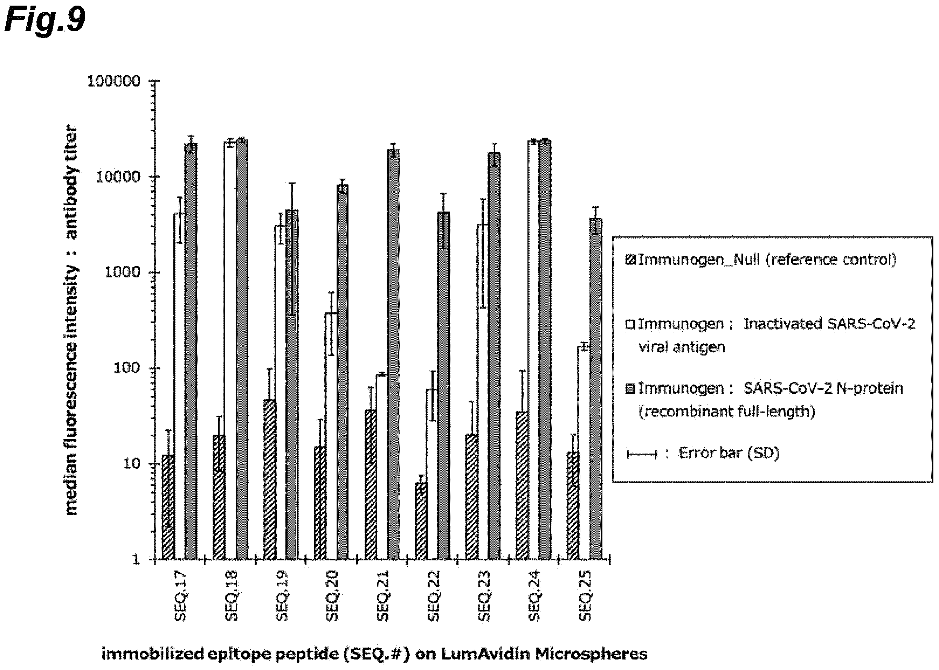

The results are shown in Figure 9. Error bars (SD) in the figure are the differences between measurements (n = 3).

As shown in Figure 9, it was confirmed that the antiserums obtained in Example 3 and Example 4 had dominant antibody titers against all the epitope sequences of the N-protein having SEQ ID NOS: 17 to 25.

## Claims

1. A monoclonal antibody or an antigen-binding fragment thereof, wherein the monoclonal antibody or the antigen-binding fragment thereof specifically reacts with a structural protein of SARS-CoV-2, and the structural protein of SARS-CoV-2 is at least one protein selected from the group consisting of S-protein, N-protein, M-protein, and E-protein.

2. The monoclonal antibody or the antigen-binding fragment thereof according to claim 1, wherein the structural protein of SARS-CoV-2 is S-protein, and the monoclonal antibody or the antigen-binding fragment thereof binds to an amino acid region of the S-protein corresponding to amino acid positions from 66 to 85 (SEQ ID NO: 5), from 87 to 107 (SEQ ID NO: 6), from 105 to 131 (SEQ ID NO: 7), from 140 to 157 (SEQ ID NO: 8), from 155 to 170 (SEQ ID NO: 9), from 522 to 541 (SEQ ID NO: 10), from 538 to 562 (SEQ ID NO: 11), from 595 to 611 (SEQ ID NO: 12), from 670 to 693 (SEQ ID NO: 13), from 761 to 797 (SEQ ID NO: 14), from 802 to 818 (SEQ ID NO: 15), or from 1235 to 1273 (SEQ ID NO: 16).

3. The monoclonal antibody or the antigen-binding fragment thereof according to claim 1 or 2, wherein the monoclonal antibody or the antigen-binding fragment thereof binds to an amino acid region of the S-protein corresponding to amino acid positions from 71 to 83, from 89 to 103, from 107 to 127, from 143 to 154, from 158 to 167, from 524 to 538, from 546 to 560, from 598 to 608, from 672 to 691, from 772 to 796, from 806 to 816, or from 1238 to 1269.

4. The monoclonal antibody or the antigen-binding fragment thereof according to claim 1 or 2, wherein the monoclonal antibody or the antigen-binding fragment thereof binds to an amino acid region of the S-protein corresponding to amino acid positions from 73 to 79, from 93 to 99, from 109 to 115, from 145 to 152, from 160 to 165, from 526 to 531, from 553 to 558, from 599 to 607, from 674 to 684, from 773 to 795, from 808 to 814, or from 1257 to 1261.

5. The monoclonal antibody or the antigen-binding fragment thereof according to claim 1, wherein the structural protein of SARS-CoV-2 is N-protein, and the monoclonal antibody or the antigen-binding fragment thereof binds to an amino acid region of the N-protein corresponding to amino acid positions from 1 to 18 (SEQ ID NO: 17), from 19 to 49 (SEQ ID NO: 18), from 174 to 207 (SEQ ID NO: 19), from 230 to 252 (SEQ ID NO: 20), from 247 to 267 (SEQ ID NO: 21), from 336 to 350 (SEQ ID NO: 22), from 362 to 379 (SEQ ID NO: 23), from 377 to 395 (SEQ ID NO: 24), or from 401 to 419 (SEQ ID NO: 25).

6. The monoclonal antibody or the antigen-binding fragment thereof according to claim 1 or 5, wherein the monoclonal antibody or the antigen-binding fragment thereof binds to an amino acid region of the N-protein corresponding to amino acid positions from 1 to 12, from 20 to 48, from 175 to 206, from 234 to 250, from 254 to 263, from 338 to 348, from 366 to 377, from 381 to 391, or from 403 to 417.

7. The monoclonal antibody or the antigen-binding fragment thereof according to claim 1 or 5, wherein the monoclonal antibody or the antigen-binding fragment thereof binds to an amino acid region of the N-protein corresponding to amino acid positions from 4 to 11, from 27 to 41, from 185 to 196, from 237 to 247, from 256 to 261, from 340 to 347, from 367 to 375, from 384 to 390, or from 406 to 415.

8. The monoclonal antibody or the antigen-binding fragment thereof according to claim 1, wherein the structural protein of SARS-CoV-2 is M-protein, and the monoclonal antibody or the antigen-binding fragment thereof binds to an amino acid region of the M-protein corresponding to amino acid positions from 1 to 24 (SEQ ID NO: 26), from 33 to 50 (SEQ ID NO: 27), from 101 to 120 (SEQ ID NO: 28), from 121 to 139 (SEQ ID NO: 29), from 146 to 171 (SEQ ID NO: 30), from 169 to 195 (SEQ ID NO: 31), or from 193 to 222 (SEQ ID NO: 32).

9. The monoclonal antibody or the antigen-binding fragment thereof according to claim 1 or 8, wherein the monoclonal antibody or the antigen-binding fragment thereof binds to an amino acid region of the M-protein corresponding to amino acid positions from 1 to 19, from 38 to 45, from 103 to 118, from 122 to 138, from 155 to 168, from 172 to 195, or from 196 to 216.

10. The monoclonal antibody or the antigen-binding fragment thereof according to claim 1 or 8, wherein the monoclonal antibody or the antigen-binding fragment thereof binds to an amino acid region of the M-protein corresponding to amino acid positions from 1 to 16, from 40 to 44, from 105 to 117, from 123 to 136, from 160 to 167, from 173 to 179, or from 205 to 215.

11. The monoclonal antibody or the antigen-binding fragment thereof according to claim 1, wherein the structural protein of SARS-CoV-2 is E-protein, and the monoclonal antibody or the antigen-binding fragment thereof binds to an amino acid region of the E-protein corresponding to amino acid positions from 1 to 19 (SEQ ID NO: 33) or from 50 to 75 (SEQ ID NO: 34).

12. The monoclonal antibody or the antigen-binding fragment thereof according to claim 1 or 11, wherein the monoclonal antibody or the antigen-binding fragment thereof binds to an amino acid region of the E-protein corresponding to amino acid positions from 4 to 13 or from 51 to 72.

13. The monoclonal antibody or the antigen-binding fragment thereof according to claim 1 or 11, wherein the monoclonal antibody or the antigen-binding fragment thereof binds to an amino acid region of the E-protein corresponding to amino acid positions from 5 to 10 or from 60 to 71.

14. A method for detecting SARS-CoV-2 in a specimen, comprising:
contacting the monoclonal antibody or the antigen-binding fragment thereof according to any one of claims 1 to 13 with the specimen and detecting SARS-CoV-2 by an immunological measuring method.

15. The method according to claim 14, wherein at least two monoclonal antibodies or antigen-binding fragments thereof being able to simultaneously bind to amino acid regions of the S-protein selected from the group consisting of amino acid regions corresponding to amino acid positions from 66 to 85 (SEQ ID NO: 5), from 87 to 107 (SEQ ID NO: 6), from 105 to 131 (SEQ ID NO: 7), from 140 to 157 (SEQ ID NO: 8), from 155 to 170 (SEQ ID NO: 9), from 522 to 541 (SEQ ID NO: 10), from 538 to 562 (SEQ ID NO: 11), from 595 to 611 (SEQ ID NO: 12), from 670 to 693 (SEQ ID NO: 13), from 761 to 797 (SEQ ID NO: 14), from 802 to 818 (SEQ ID NO: 15), and from 1235 to 1273 (SEQ ID NO: 16) are used.

16. The method according to claim 14, wherein at least two monoclonal antibodies or antigen-binding fragments thereof being able to simultaneously bind to amino acid regions of the N-protein selected from the group consisting of amino acid regions corresponding to amino acid positions from 1 to 18 (SEQ ID NO: 17), from 19 to 49 (SEQ ID NO: 18), from 174 to 207 (SEQ ID NO: 19), from 230 to 252 (SEQ ID NO: 20), from 247 to 267 (SEQ ID NO: 21), from 336 to 350 (SEQ ID NO: 22), from 362 to 379 (SEQ ID NO: 23), from 377 to 395 (SEQ ID NO: 24), and from 401 to 419 (SEQ ID NO: 25) are used.

17. The method according to claim 14, wherein at least two monoclonal antibodies or antigen-binding fragments thereof being able to simultaneously bind to amino acid regions of the M-protein selected from the group consisting of amino acid regions corresponding to amino acid positions from 1 to 24 (SEQ ID NO: 26), from 33 to 50 (SEQ ID NO: 27), from 101 to 120 (SEQ ID NO: 28), from 121 to 139 (SEQ ID NO: 29), from 146 to 171 (SEQ ID NO: 30), from 169 to 195 (SEQ ID NO: 31), and from 193 to 222 (SEQ ID NO: 32) are used.

18. The method according to claim 14, wherein two monoclonal antibodies or antigen-binding fragments thereof being able to simultaneously bind to amino acid regions of the E-protein corresponding to amino acid positions from 1 to 19 (SEQ ID NO: 33) and from 50 to 75 (SEQ ID NO: 34) are used.

19. A detection kit of SARS-CoV-2, comprising:
the monoclonal antibody or the antigen-binding fragment thereof according to any one of claims 1 to 13.

20. The kit according to claim 19, comprising:
at least two monoclonal antibodies or antigen-binding fragments thereof being able to simultaneously bind to amino acid regions of the S-protein selected from the group consisting of amino acid regions corresponding to amino acid positions from 66 to 85 (SEQ ID NO: 5), from 87 to 107 (SEQ ID NO: 6), from 105 to 131 (SEQ ID NO: 7), from 140 to 157 (SEQ ID NO: 8), from 155 to 170 (SEQ ID NO: 9), from 522 to 541 (SEQ ID NO: 10), from 538 to 562 (SEQ ID NO: 11), from 595 to 611 (SEQ ID NO: 12), from 670 to 693 (SEQ ID NO: 13), from 761 to 797 (SEQ ID NO: 14), from 802 to 818 (SEQ ID NO: 15), and from 1235 to 1273 (SEQ ID NO: 16).

21. The kit according to claim 19, comprising:
at least two monoclonal antibodies or antigen-binding fragments thereof according to claim 5 being able to simultaneously bind to amino acid regions of the N-protein selected from the group consisting of amino acid regions corresponding to amino acid positions from 1 to 18 (SEQ ID NO: 17), from 19 to 49 (SEQ ID NO: 18), from 174 to 207 (SEQ ID NO: 19), from 230 to 252 (SEQ ID NO: 20), from 247 to 267 (SEQ ID NO: 21), from 336 to 350 (SEQ ID NO: 22), from 362 to 379 (SEQ ID NO: 23), from 377 to 395 (SEQ ID NO: 24), and from 401 to 419 (SEQ ID NO: 25).

22. The kit according to claim 19, comprising:
at least two monoclonal antibodies or antigen-binding fragments thereof being able to simultaneously bind to amino acid regions of the M-protein selected from the group consisting of amino acid regions corresponding to amino acid positions from 1 to 24 (SEQ ID NO: 26), from 33 to 50 (SEQ ID NO: 27), from 101 to 120 (SEQ ID NO: 28), from 121 to 139 (SEQ ID NO: 29), from 146 to 171 (SEQ ID NO: 30), from 169 to 195 (SEQ ID NO: 31), and from 193 to 222 (SEQ ID NO: 32).

23. The kit according to claim 19, comprising:
two monoclonal antibodies or antigen-binding fragments thereof being able to simultaneously bind to amino acid regions of the E-protein corresponding to amino acid positions from 1 to 19 (SEQ ID NO: 33) and from 50 to 75 (SEQ ID NO: 34).

24. A monoclonal antibody or an antigen-binding fragment thereof, wherein the monoclonal antibody or the antigen-binding fragment thereof specifically reacts with a structural protein of SARS-CoV-2 and a structural protein of a human infectious coronavirus other than SARS-CoV-2, the structural protein of SARS-CoV-2 is at least one selected from the group consisting of S-protein, N-protein, M-protein, and E-protein, and the structural protein of the human infectious coronavirus other than SARS-CoV-2 is at least one selected from the group consisting of S-protein, N-protein, M-protein, and E-protein.

25. The monoclonal antibody or the antigen-binding fragment thereof according to claim 24, wherein the human infectious coronavirus other than SARS-CoV-2 is MERS-CoV and/or SARS-CoV.

26. A method for detecting SARS-CoV-2 and a human infectious coronavirus other than SARS-CoV-2 in a specimen, comprising:
contacting the monoclonal antibody or the antigen-binding fragment thereof according to claim 24 or 25 with the specimen and
detecting SARS-CoV-2 and the human infectious coronavirus other than SARS-CoV-2 by an immunological measuring method.

27. A detection kit for SARS-CoV-2 and a human infectious coronavirus other than SARS-CoV-2, comprising:
the monoclonal antibody or the antigen-binding fragment thereof according to claim 24 or 25.

28. A polypeptide, comprising:
any one amino acid sequence of SEQ ID NOS: 5 to 34.

29. A method for detecting an anti-SARS-CoV-2 antibody in a specimen, comprising:
contacting the polypeptide according to claim 28 with the specimen and detecting the anti-SARS-CoV-2 antibody by an immunological measuring method.

30. A detection kit for an anti-SARS-CoV-2 antibody, comprising:
the polypeptide according to claim 28.

31. A method for detecting an anti-SARS-CoV-2 antibody and an anti-human infectious coronavirus antibody other than the anti-SARS-CoV-2 antibody in a specimen, comprising:
contacting the polypeptide according to claim 28 with the specimen and detecting the anti-SARS-CoV-2 antibody and the anti-human infectious coronavirus antibody other than the anti-SARS-CoV-2 antibody by an immunological measuring method.

32. A detection kit for an anti-SARS-CoV-2 antibody and an anti-human infectious coronavirus antibody other than the anti-SARS-CoV-2 antibody, comprising:
the polypeptide according to claim 28.

33. A vaccine for SARS-CoV-2, comprising:
the polypeptide according to claim 28.

34. A vaccine for SARS-CoV-2 and a human infectious coronavirus other than SARS-CoV-2, comprising:
the polypeptide according to claim 28.

35. An antibody drug for treating a SARS-CoV-2 infection, comprising:
a monoclonal antibody or an antigen-binding fragment specifically reacting with the polypeptide according to claim 28.

36. An antibody drug for treating a SARS-CoV-2 infection and an infection due to a human infectious coronavirus other than SARS-CoV-2, comprising:
a monoclonal antibody or an antigen-binding fragment specifically reacting with the polypeptide according to claim 28.
